# EUROPEAN PATENT SPECIFICATION

(11) **EP 4 054 481 B1**
(45) Date of publication and mention of the grant of the patent: **08.04.2026**
(21) Application number: 20884797.0
(22) Date of filing: 06.11.2020
(51) Int. Cl.: A61F 2/07, A61F 2/06

(54) **ENDOGRAFT WITH BRISTLES**
ENDOGRAFT MIT BORSTEN
ENDOGREFFE À POILS

(30) Priority: 07.11.2019 US 201962932186 P
(43) Date of publication of application: 14.09.2022
(73) Proprietor: Madjarov, Jeko Metodiev, Wilmington, NC 28405 (US)
(72) Inventor: Madjarov, Jeko Metodiev, Wilmington, NC 28405 (US)
(74) Representative: Papula Oy
(86) International application number: PCT/US2020/059498
(87) International publication number: WO 2021/092453

(56) References cited:
- EP-A1- 3 459 496
- WO-A2-01/66167
- FR-A1- 2 991 162
- US-A1- 2004 186 557
- US-A1- 2005 143 806
- US-A1- 2009 287 297
- US-A1- 2012 123 511
- US-A1- 2015 297 252
- US-A1- 2016 030 047
- US-A1- 2016 030 209
- US-A1- 2019 167 411

## Description

### CROSS-REFERENCE TO RELATED APPLICATION

The present application claims the benefit of priority of U.S. Provisional Patent Application No. 62/932,186 filed on November 7, 2019.

### FIELD

The presently-disclosed invention relates generally to vascular endografts and methods of making and using the same, and more particularly to vascular endografts providing improved blood turbulence and clotting while minimizing endoleaks and methods of making and using the same.

### BACKGROUND

The traditional repair of abnormal bulging (aneurysm) of blood vessels is to resect (remove) the diseased portion and replace it with a synthetic vascular graft. Because this usually requires opening of a body cavity and performing a major operation with considerable surgical trauma, in recent decades the technique of endovascular repair (EVAR) has been developed. In the course of EVAR a fabric-covered stent-graft is put in place using a special catheter, inserted through a puncture-hole on the skin and put in place under X-ray control.

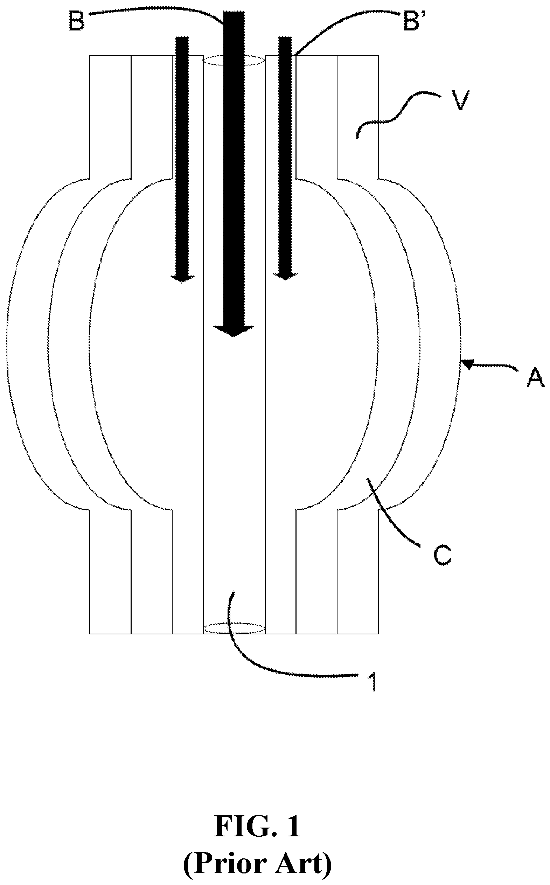

The endograft reinforces the area of the vessel (usually the aorta) which is weakened due to an aneurysm. FIG. 1 illustrates a conventional endograft 1 positioned in a vessel V exhibiting aneurysm A. The endograft 1 may also be applied in areas which are not dilated but weakened by other pathology, including dissection. The endograft 1 provides a path for the blood flow B and at the same time is intended to prevent the blood from reaching, and the blood pressure from rupturing, the weakened portion of the vessel wall. After implantation, a dead-space is present between the endograft 1 and the wall of the aneurysm. This dead-space is usually filled with the stagnant blood forming a clot C, encapsulated between the area between the endograft 1 and the wall of the vessel. Similarly, if the endograft 1 is placed in a vessel segment with irregular inside layer portions of ulcerations or other irregularities, those irregularities are expected to be excluded from circulation by EVAR and filled with clotted blood or fibrin. Eventually, the uneven surfaces promote the presence of platelets and in general clotting. Fibrosis ensues later. In this way, the aneurysmal sac surrounding portions of the endograft are eliminated, which promotes positive remodeling.

The principal complication of EVAR is endoleak, *i.e.* some amount of blood flow (labeled B' in FIG. 1) still reaches the area between the endograft and the vessel wall. This can occur either around the fixation sites (or landing zones) or by collateral flow, which may reach the aneurysm from tributaries. Such a situation may prevent the formation of an occlusive clot and could lead to rupture of the aorta or other serious complications. The surface of the conventional endograft 1 facing the excluded portion of the aorta is smooth, which is unfavorable to clot formation.

Accordingly, there still exists a need for an endograft that minimizes endoleaks and/or encourages blood turbulence, clotting, and/or positive remodeling of the vessel wall. As an example, WO 01/66167 A2 discloses an endovascular stent for vascular vessels which can be used to occlude the vessel or which can be used to bridge damaged areas in the vessel.

### BRIEF SUMMARY

One or more embodiments of the invention may address one or more of the aforementioned problems. Certain embodiments according to the invention provide vascular endografts that minimize endoleaks and/or encourage blood turbulence, clotting, and/or positive remodeling of the vessel wall. In particular, according to a first aspect of the invention, an endograft is provided, the endograft being configured to be positioned within a diseased portion of a blood vessel. The endograft comprises an elongated body defining a lumen configured to allow blood to flow therethrough and further defining an exterior surface, and a plurality of bristles extending outwardly from the exterior surface of the elongated body, the plurality of bristles comprising nitinol and being configured to curl into a preconfigured non-linear shape when warmed to a body temperature. In an instance in which the endograft is positioned within a blood vessel at a location corresponding to a blood vessel pathology, the bristles encourage positive remodeling of the blood vessel.

According to certain embodiments, the elongated body may comprise a mesh frame covered in fabric. In some embodiments, the mesh frame may comprise nitinol. In further embodiments, the fabric may comprise expanded polytetrafluoroethylene.

According to certain embodiments, the plurality of bristles may be integral to the exterior surface of the elongated body. In other embodiments, the plurality of bristles may be attached to the elongated body.

According to certain embodiments, the elongated body may define a first end and a second end, and the plurality of bristles may extend outwardly from the exterior surface of the elongated body from the first end to the second end of the elongated body. In other embodiments, the elongated body may define a first end and a second end, and the plurality of bristles may extend outwardly from the exterior surface of the elongated body only proximate the first end and the second end of the elongated body.

According to certain embodiments, the plurality of bristles may comprise bristles of unequal lengths.

According to certain embodiments, the blood vessel may be an aorta. In some embodiments, the blood vessel pathology may be an aneurysm. In other embodiments, the blood vessel pathology may be an aortic dissection.

According to certain embodiments, the endograft may be a branched endograft, and the elongated body may define at least a first lumen and a second lumen.

In a second aspect of the invention, a method of encouraging positive remodeling of a diseased portion of a blood vessel is provided. The method comprises positioning an endograft within the diseased portion of the blood vessel. The endograft comprises an elongated body defining a lumen configured to allow blood to flow therethrough and further defining an exterior surface, and a plurality of bristles extending outwardly from the exterior surface of the elongated body, the plurality of bristles comprising nitinol and being configured to curl into a preconfigured non-linear shape when warmed to a body temperature. The plurality of bristles is configured to facilitate clotting of blood flowing thereby.

According to certain embodiments, positioning the endograft within the diseased portion of the blood vessel may comprise positioning the endograft within an aortic dissection. In other embodiments, positioning the endograft within the diseased portion of the blood vessel may comprise positioning the endograft within an aneurysm.

In a third aspect of the invention, a method of making an endograft is provided. The method comprises forming an elongated body defining a lumen configured to allow blood to flow therethrough, and providing a plurality of bristles extending outwardly from the exterior surface of the elongated body. The plurality of bristles is configured to facilitate clotting of blood flowing thereby to encourage positive remodeling of a diseased portion of a blood vessel within which the endograft is positioned.

According to certain embodiments, providing the plurality of bristles may comprise forming the plurality of bristles integrally with the elongated body. In other embodiments, providing the plurality of bristles may comprise forming the plurality of bristles separately from the elongated body and attaching the plurality of bristles to the elongated body.

### BRIEF DESCRIPTION OF THE SEVERAL VIEWS OF THE DRAWING(S)

Having thus described the invention in general terms, reference will now be made to the accompanying drawings, which are not necessarily drawn to scale, and wherein:
FIG. 1 illustrates a prior art endograft implanted into a blood vessel with an aneurysm;
FIG. 2 is a side view of an endograft in accordance with certain embodiments of the invention;
FIG. 3 is an end view of an endograft in accordance with certain embodiments of the invention;
FIG. 4 illustrates an endograft implanted in a blood vessel in accordance with certain embodiments of the invention;
FIG. 5 illustrates an endograft implanted into a blood vessel with an aneurysm in accordance with certain embodiments of the invention;
FIG. 6 illustrates endografts implanted in an aortic dissection in accordance with certain embodiments of the invention; and
FIG. 7 illustrates a branched endograft in accordance with certain embodiments of the invention.

### DETAILED DESCRIPTION

The invention now will be described more fully hereinafter with reference to the accompanying drawings, in which some, but not all embodiments of the inventions are shown. Indeed, this invention may be embodied in many different forms and should not be construed as limited to the embodiments set forth herein; rather, these embodiments are provided so that this disclosure will satisfy applicable legal requirements. Like numbers refer to like elements throughout. As used in the specification, and in the appended claims, the singular forms "a", "an", "the", include plural referents unless the context clearly dictates otherwise.

The invention includes, according to certain embodiments, vascular endografts providing improved blood turbulence and clotting while preventing endoleaks. In particular, according to a first aspect of the invention, an endograft is provided, the endograft being configured to be positioned within a blood vessel in a location corresponding to a diseased portion of the blood vessel. The endograft comprises an elongated body defining a lumen configured to allow blood to flow therethrough and further defining an exterior surface, and a plurality of bristles extending outwardly from the exterior surface of the elongated body, the plurality of bristles comprising nitinol and being configured to curl into a preconfigured non-linear shape when warmed to a body temperature. In an instance in which the endograft is positioned within a blood vessel at a location corresponding to a blood vessel pathology, the bristles encourage positive remodeling of the blood vessel.

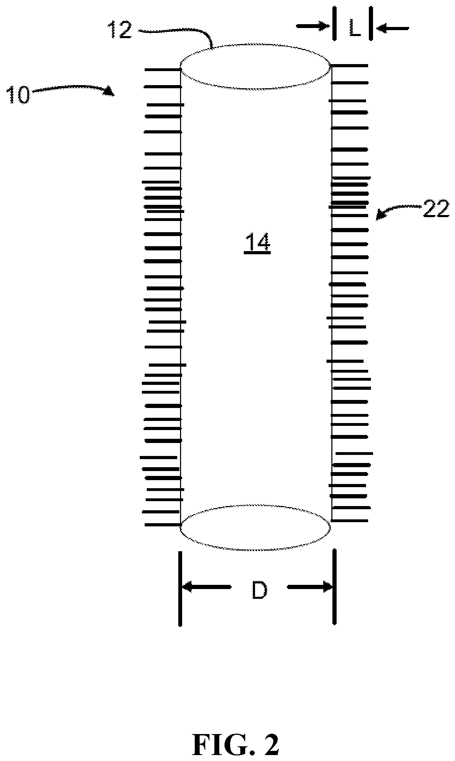

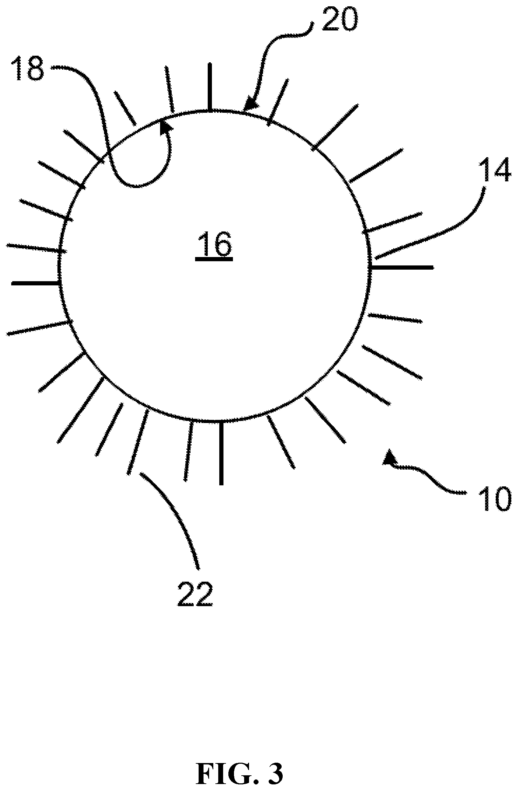

Turning to the figures, FIGs. 2 and 3 illustrate an exemplary vascular endograft 10. In general, the endograft 10 may have an elongated body 12. The body 12 may be formed by a closed peripheral wall 14 defining an internal bore 16 which may be circular or some other convenient cross-sectional shape. The peripheral wall 14 may have opposed interior and exterior surfaces 18, 20 respectively. The peripheral wall 14 may be homogenous or may be made of multiple layers of the same or different materials.

In accordance with certain embodiments, the size (e.g. inside diameter "D") of the body 12 may be selected to suit a particular application and depends principally upon the size of the blood vessel to be repaired. An example range of diameters is from about 6 mm to about 50 mm.

According to certain embodiments, the body 12 may comprise a mesh frame covered in fabric. In some embodiments, for example, the body 12 may be formed from a biocompatible material, that is, a material that is not harmful to living tissue. Known biocompatible materials include some polymers and metal alloys. The construction may be, for example, extruded, molded, woven, or knitted. In some embodiments, for instance, the mesh frame may comprise nitinol. In further embodiments, for example, the fabric may comprise expanded polytetrafluoroethylene (ePTFE), also known as GORE-TEX^{®}.

A plurality of bristles 22 may extend outwardly from the exterior surface 20 of the peripheral wall 14. These bristles 22 may allow less radial force to be required to keep the endograft in place, thereby resulting in less stress on the native aortic wall and landing zones. The bristles 22 may be small, hair-like structures. In general, the greater the diameter of the body 12, the longer the bristles 22 should be. An example range for the length "L" of the bristles may be from about 2 mm to about 4 mm.

The bristles 22 need not have any particular stiffness and may be highly flexible so as to not apply undue pressure on the vessel and to be able to flex with blood flow. As an example, the bristles 22 may have a thickness on the order of about 0.1 to about 0.5 mm.

The bristles 22 may be disposed uniformly or non-uniformly over the exterior surface 20. In some embodiments, for example those directed to treating aneurysms, the bristles 22 may be disposed on the exterior surface 20 from a first end to a second end of the elongated body 12, such that the bristles cover the entire length (or nearly the entire length) of the body. In other embodiments, for instance, the bristles 22 may be provided only on a portion of the exterior surface 20. For example, a short band of bristles 22 may be provided around each distal end of the body 12, e.g., only in the regions corresponding to the landing zones, such as in embodiments directed to treating aortic dissection. Optionally, the bristles 22 may have different lengths, diameters, and/or material compositions in different areas of the exterior surface 20. For instance, the plurality of bristles 22 may have unequal lengths. Generally, the bristles 22 should be densely spaced. For example, the inter-bristle spacing should be less than the bristle length L and may be much closer.

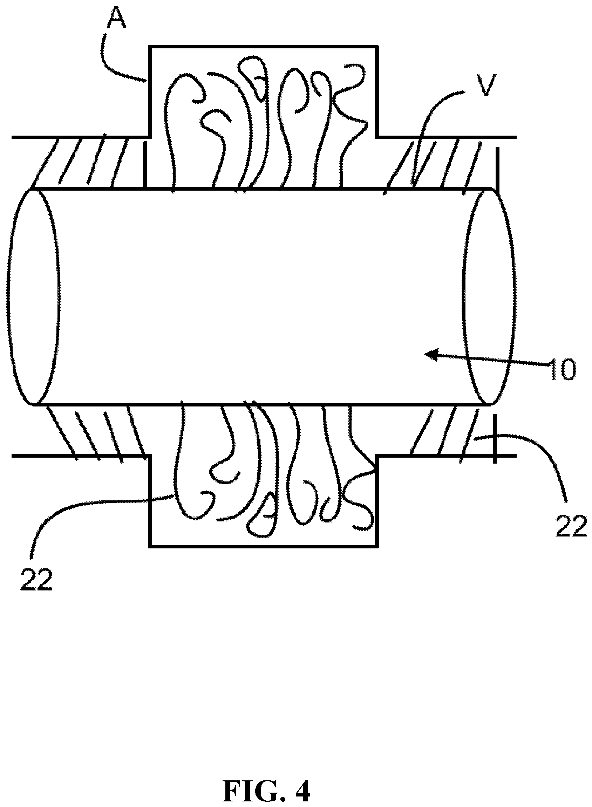

The bristles 22 may be formed from a biocompatible material that may be the same or different from the material of the peripheral wall 14. For example, in some embodiments, the plurality of bristles 22 may comprise nitinol, ePTFE (GORE-TEX^{®}), polytetrafluoroethylene (PTFE), also known as TEFLON^{®}, or stainless steel. The bristles 22 comprising nitinol are formed in a preconfigured temperature-activated shape such that the bristles 22 are straight until the endograft is deployed in the body and the bristles warm up to body temperature, at which point they will curl up into their preconfigured non-linear shape to further create blood turbulence. An example of an endograft 10 with bristles 22 in at least one area of the elongated body (e.g., the mid-portion) formed so as to have a preconfigured non-linear shape when deployed is shown in FIG. 4. As such, in some embodiments, some of the bristles may be made using one type of material, and other bristles may be made using another type of material. Similarly, some bristles may have one configuration when deployed (e.g., linear) and some bristles may have another configuration when deployed (e.g., non-linear).

The bristles 22 are securely connected at their proximal ends to the peripheral wall 14. The bristles 22 may be made separately and attached to the peripheral wall 14 or alternatively they may be formed integrally with the peripheral wall 14.

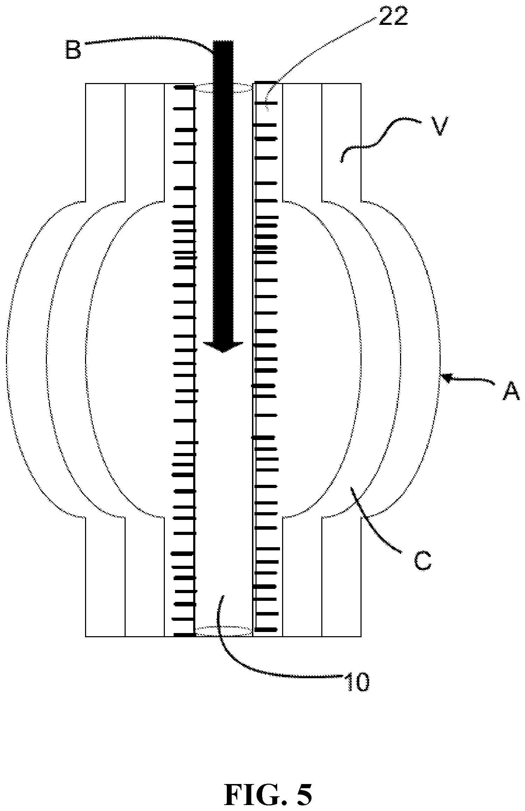

FIGs. 4 and 5 illustrate the endograft 10 implanted in a vessel V (depicted schematically). In some cases, the bristles 22 may lay flat against the vessel wall if the endograft 10 fills the entire vessel lumen or stand erect if the vessel V is dilated beyond the caliber of the EVAR (e.g., due to aneurysm A). In other cases, such as in the depicted embodiment of FIG. 4, a length of the bristles 22 in the area of the landing zones (e.g., proximate the first and second ends of the elongated body) may be shorter than a length of the bristles in the mid-portion of the endograft 10. The bristles 22 in the mid-portion may have a longer length to allow the bristles to extend farther toward the wall of the blood vessel in the area of the aneurysm A to provide more turbulence to the blood flow within the aneurysm. In other embodiments (not shown), longer and shorter bristles may be interspersed along the exterior of the elongated body, such as in the mid-portion of the endograft 10 in the area of the aneurysm A and/or proximate the first and second ends of the elongated body.

Regardless of the relative length and location of the bristles 22, the bristles 22 create an uneven surface for the blood to flow past, creating more turbulent blood flow within the expanded area of the aneurysm A. As a result, the chances of forming a clot between the endograft 10 and the vessel wall are significantly enhanced. Moreover, the formation of such clots in the area of the aneurysm A encourages positive remodeling of the blood vessel, thereby shrinking the size of the aneurysm. Such clotting further reduces the risk of transgraft leakage, or leakage through the wall of the endograft 10 and into the aneurysm A.

For similar reasons, in the vicinity of the proximal and distal ends of the endograft 10, such clotting reduces the risk of endoleakage (e.g., the flow of blood around the exterior of the endograft and into the aneurysm). If an endoleak occurs, the bristles 22 proximate the proximal and distal ends of the endograft promote late occlusion by the same clotting mechanism. Such clotting in the landing zones (e.g., proximate the proximal and distal ends of the endograft) further enhances the stability of the endograft within the blood vessel by more securely fixing the endograft to the blood vessel wall.

According to certain embodiments, the blood vessel may be the aorta. In some embodiments, for instance, the blood vessel pathology may be an aneurysm, as illustrated in FIG. 5 and discussed above.

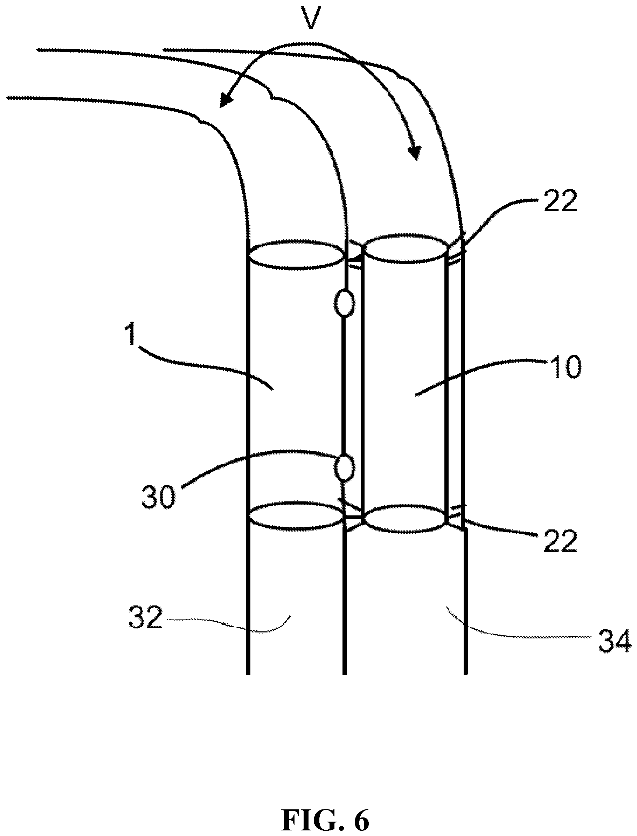

In other embodiments, the blood vessel pathology may be an aortic dissection. In such embodiments, and as illustrated in FIG. 6, two endografts may be used, with one endograft positioned in the true lumen 32 and one endograft positioned in the false lumen 34. In some cases, each of the two endografts may be configured as an endograft 10 according to the embodiments described above, such that both have bristles 22, whereas in other cases only one of the endografts may be configured as an endograft 10 having bristles 22. If only one endograft has bristles 22, as shown in FIG. 6, then an endograft 10 having bristles 22 according to embodiments of the invention may be disposed in the false lumen 34. In certain embodiments, the bristles 22 may be positioned on the body 12 only in the landing zones proximate the first and second ends of the elongated body 12 of the endograft 10 to avoid blood leakage around the endograft. In some embodiments, for instance, the wall of the blood vessel in the area of the dissection, such as the wall between the true lumen and the false lumen 34, may include one or more fenestrations 30 or openings. As such, minimized endoleakage due to the presence of the bristles near the first and second ends of the elongated body 12 of the endograft 10 serves to minimize the flow of blood from the false lumen into the true lumen via the fenestrations and to keep blood flowing through the false lumen. In this way, blood flow through the false lumen may be maintained to allow the blood to reach corresponding arteries of the visceral segment of the aorta, so as to maintain perfusion in the distal abdominal aorta and peripheral circulation.

In addition, the enhanced clotting due to the presence of the bristles 22 proximate the first and second ends of the elongated body 12 serves to more securely fix the position of the endograft within the blood vessel without the need for excessive radial force to be applied by the endograft. The reduction of radial force in the false lumen is especially beneficial to reduce stress on the wall of the aorta, given the diseased and weakened nature of the vessel wall due to the dissection.

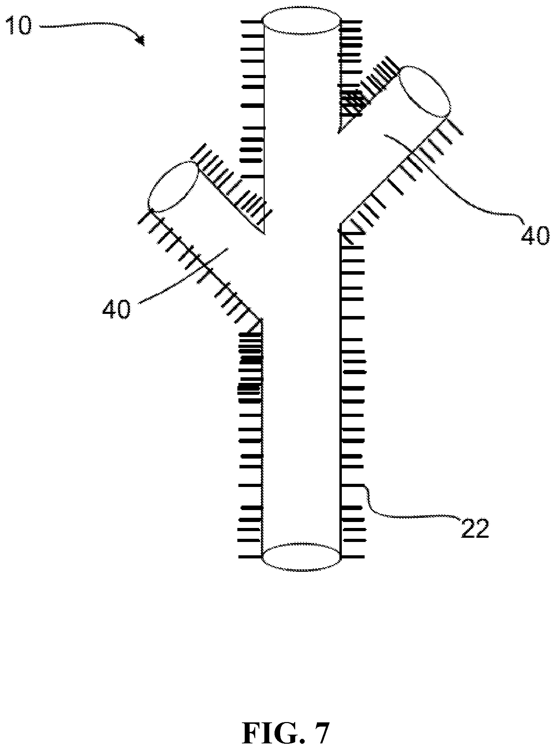

In accordance with certain embodiments, and as shown in FIG. 7, the endograft may be a branched endograft. In such embodiments, for example, the elongated body may define at least a first lumen and a second lumen. For instance, as shown in FIG. 7, the endograft 10 may have more than two lumens 40 corresponding to branching of the blood vessels in the location of the patient's body to be treated. Such branched endografts are described in more detail in U.S. Patent No. 10,219 890**.** In certain embodiments, for example, an exterior of the elongated body in the area of the endograft that defines at least one of the lumens 40 may comprise bristles 22.

In a second aspect of the invention, a method of encouraging positive remodeling of a diseased portion of a blood vessel (e.g., shrinking of the vessel wall towards the endograft) is provided. The method comprises positioning an endograft within the diseased portion of the blood vessel. As discussed previously herein, the endograft comprises an elongated body defining a lumen configured to allow blood to flow therethrough and further defining an exterior surface, and a plurality of bristles extending outwardly from the exterior surface of the elongated body. The plurality of bristles is configured to facilitate clotting of blood flowing thereby, as described above with reference to the figures.

According to certain embodiments, positioning the endograft within the diseased portion of the blood vessel may comprise positioning the endograft in a location of an aortic dissection. In other embodiments, positioning the endograft within the diseased portion of the blood vessel may comprise positioning the endograft in a location of an aneurysm.

In a third aspect of the invention, a method of making an endograft is provided. The method comprises forming an elongated body defining a lumen configured to allow blood to flow therethrough, and providing a plurality of bristles extending outwardly from the exterior surface of the elongated body. The plurality of bristles is configured to facilitate clotting of blood flowing thereby to encourage positive remodeling of a diseased portion of a blood vessel within which the endograft is positioned.

According to certain embodiments, providing the plurality of bristles may comprise forming the plurality of bristles integrally with the elongated body. In other embodiments, providing the plurality of bristles may comprise forming the plurality of bristles separately from the elongated body and attaching the plurality of bristles to the elongated body.

Modifications of the invention set forth herein will come to mind to one skilled in the art to which the invention pertains having the benefit of the teachings presented in the foregoing descriptions and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation. The invention is disclosed in the appended claims.

## Claims

1. An endograft (10) configured to be positioned within a blood vessel in a location corresponding to a diseased portion of the blood vessel, the endograft comprising:
an elongated body (12) defining a lumen configured to allow blood to flow therethrough and further defining an exterior surface (20); and
a plurality of bristles (22) extending outwardly from the exterior surface of the elongated body,
wherein, in an instance in which the endograft is positioned within a blood vessel at a location corresponding to a blood vessel pathology, the bristles encourage positive remodeling of the blood vessel;
**characterized by** the plurality of bristles comprising nitinol and being configured to curl into a preconfigured non-linear shape when warmed to a body temperature.

2. The endograft of Claim 1, wherein the elongated body comprises a mesh frame covered in fabric.

3. The endograft of Claim 2, wherein the mesh frame comprises nitinol.

4. The endograft of Claim 2, wherein the fabric comprises expanded polytetrafluoroethylene.

5. The endograft of Claim 1, wherein the plurality of bristles is integral to the exterior surface of the elongated body.

6. The endograft of Claim 1, wherein the plurality of bristles is attached to the elongated body.

7. The endograft of Claim 1, wherein the elongated body defines a first end and a second end, wherein the plurality of bristles extends outwardly from the exterior surface of the elongated body from the first end to the second end of the elongated body.

8. The endograft of Claim 1, wherein the elongated body defines a first end and a second end, wherein the plurality of bristles extends outwardly from the exterior surface of the elongated body only proximate the first end and the second end of the elongated body.

9. The endograft of Claim 1, wherein the plurality of bristles comprises bristles of unequal lengths.

10. A method of making an endograft, the method comprising:
forming an elongated body defining a lumen configured to allow blood to flow therethrough; and
providing a plurality of bristles extending outwardly from the exterior surface of the elongated body,
wherein the plurality of bristles is configured to facilitate clotting of blood flowing thereby to encourage positive remodeling of a diseased portion of a blood vessel in a location at which the endograft is positioned;
**characterized by** the plurality of bristles comprising nitinol and being configured to curl into a preconfigured non-linear shape when warmed to a body temperature.

11. The method of Claim 10, wherein providing the plurality of bristles comprises forming the plurality of bristles integrally with the elongated body.

12. The method of Claim 10, wherein providing the plurality of bristles comprises forming the plurality of bristles separately from the elongated body and attaching the plurality of bristles to the elongated body.

## Patentansprüche

1. Endograft (10), das dazu konfiguriert ist, innerhalb eines Blutgefäßes an einer Position, die einem erkrankten Bereich des Blutgefäßes entspricht, positioniert zu werden, wobei das Endograft umfasst:
einen länglichen Körper (12), der ein Lumen definiert, das dazu konfiguriert ist, Blut zu erlauben, durch dieses hindurchzufließen, und ferner eine äußere Oberfläche (20) definiert; und
eine Mehrzahl von Borsten (22), die sich von der äußeren Oberfläche des länglichen Körpers nach außen erstrecken,
wobei in einem Fall, in dem das Endograft innerhalb eines Blutgefäßes an einer Position positioniert ist, die einer Blutgefäßpathologie entspricht, die Borsten eine positive Umgestaltung des Blutgefäßes fördern,
**dadurch gekennzeichnet, dass** die Mehrzahl der Borsten Nitinol umfassen und dazu konfiguriert sind, sich bei Erwärmung auf eine Körpertemperatur zu einer vorkonfigurierten nichtlinearen Form zu krümmen.

2. Endograft nach Anspruch 1, wobei der längliche Körper einen mit Stoff abgedeckten Netzrahmen umfasst.

3. Endograft nach Anspruch 2, wobei der Netzrahmen Nitinol umfasst.

4. Endograft nach Anspruch 2, wobei der Stoff expandiertes Polytetrafluorethylen umfasst.

5. Endograft nach Anspruch 1, wobei die Mehrzahl von Borsten integraler Bestandteil der äußeren Oberfläche des länglichen Körpers ist.

6. Endograft nach Anspruch 1, wobei die Mehrzahl von Borsten an dem länglichen Körper angebracht ist.

7. Endograft nach Anspruch 1, wobei der längliche Körper ein erstes Ende und ein zweites Ende definiert, wobei sich die Mehrzahl von Borsten von der äußeren Oberfläche des länglichen Körpers vom ersten Ende zum zweiten Ende des länglichen Körpers nach außen erstreckt.

8. Endograft nach Anspruch 1, wobei der längliche Körper ein erstes Ende und ein zweites Ende definiert, wobei sich die Mehrzahl von Borsten nur in der Nähe des ersten Endes und des zweiten Endes des länglichen Körpers von der äußeren Oberfläche des länglichen Körpers nach außen erstreckt.

9. Endograft nach Anspruch 1, wobei die Mehrzahl von Borsten Borsten unterschiedlicher Länge umfasst.

10. Verfahren zur Herstellung eines Endografts, wobei das Verfahren umfasst:
Bilden eines länglichen Körpers, der ein Lumen definiert, das dazu konfiguriert ist, Blut zu erlauben, durch dieses hindurchzufließen; und
Bereitstellen einer Mehrzahl von Borsten, die sich von der äußeren Oberfläche des länglichen Körpers nach außen erstrecken,
wobei die Mehrzahl von Borsten dazu konfiguriert ist, die Gerinnung des hindurchfließenden Blutes zu erleichtern, um eine positive Umgestaltung eines erkrankten Bereichs eines Blutgefäßes an einer Position zu fördern, an der das Endograft positioniert ist;
**dadurch gekennzeichnet, dass** die Mehrzahl der Borsten Nitinol umfassen und dazu konfiguriert sind, sich bei Erwärmung auf eine Körpertemperatur zu einer vorkonfigurierten nichtlinearen Form zu krümmen.

11. Verfahren nach Anspruch 10, wobei das Bereitstellen der Mehrzahl von Borsten das Bilden der Mehrzahl von Borsten integral mit dem länglichen Körper umfasst.

12. Verfahren nach Anspruch 10, wobei das Bereitstellen der Mehrzahl von Borsten das separate Bilden der Mehrzahl von Borsten vom länglichen Körper und das Anbringen der Mehrzahl von Borsten an dem länglichen Körper umfasst.

## Revendications

1. Endogreffe (10) configurée pour être positionnée à l'intérieur d'un vaisseau sanguin dans un emplacement
correspondant à une portion malade du vaisseau sanguin, l'endogreffe comprenant :
un corps allongé (12) définissant une lumière configurée pour permettre au sang de s'écouler à travers celle-ci et définissant en outre une surface extérieure (20) ; et
une pluralité de poils (22) s'étendant vers l'extérieur à partir de la surface extérieure du corps allongé,
dans laquelle, dans un cas où l'endogreffe est positionnée à l'intérieur d'un vaisseau sanguin à un emplacement correspondant à une pathologie de vaisseau sanguin, les poils favorisent un remodelage positif du vaisseau sanguin ;
**caractérisée par** la pluralité de poils comprenant du nitinol et étant configurée pour s'enrouler dans une forme préconfigurée non linéaire lorsqu'ils sont chauffés à la température corporelle.

2. Endogreffe selon la revendication 1, dans laquelle le corps allongé comprend un cadre en maille recouvert de tissu.

3. Endogreffe selon la revendication 2, dans laquelle le cadre en maille comprend du nitinol.

4. Endogreffe selon la revendication 2, dans laquelle le tissu comprend du polytétrafluoroéthylène expansé.

5. Endogreffe selon la revendication 1, dans laquelle la pluralité de poils fait partie intégrante de la surface extérieure du corps allongé.

6. Endogreffe selon la revendication 1, dans laquelle la pluralité de poils est fixée au corps allongé.

7. Endogreffe selon la revendication 1, dans laquelle le corps allongé définit une première extrémité et une seconde extrémité, dans laquelle la pluralité de poils s'étend vers l'extérieur à partir de la surface extérieure du corps allongé depuis la première extrémité vers la seconde extrémité du corps allongé.

8. Endogreffe selon la revendication 1, dans laquelle le corps allongé définit une première extrémité et une seconde extrémité, dans laquelle la pluralité de poils s'étend vers l'extérieur à partir de la surface extérieure du corps allongé uniquement à proximité de la première extrémité et de la seconde extrémité du corps allongé.

9. Endogreffe selon la revendication 1, dans laquelle la pluralité de poils comprend des soies de longueurs inégales.

10. Procédé destiné à fabriquer une endogreffe, le procédé comprenant :
former un corps allongé définissant une lumière configurée pour permettre au sang de s'écouler à travers celle-ci ; et
fournir une pluralité de poils s'étendant vers l'extérieur à partir de la surface extérieure du corps allongé,
dans laquelle la pluralité de poils est configurée pour faciliter la coagulation du sang qui s'écoule et ainsi favoriser le remodelage positif d'une portion malade d'un vaisseau sanguin dans un emplacement où l'endogreffe est positionnée ;
**caractérisée par** la pluralité de poils comprenant du nitinol et étant configurée pour s'enrouler dans une forme préconfigurée non linéaire lorsqu'ils sont chauffés à la température corporelle.

11. Procédé selon la revendication 10, dans lequel la fourniture de la pluralité de poils comprend le fait de former la pluralité de poils de manière intégrante avec le corps allongé.

12. Procédé selon la revendication 10, dans lequel la fourniture de la pluralité de poils comprend le fait de former la pluralité de poils séparément du corps allongé et de fixer la pluralité de poils au corps allongé.
